# EUROPEAN PATENT APPLICATION

(11) **EP 3 407 280 A1**
(43) Date of publication of application: **28.11.2018**
(21) Application number: 17172892.6
(22) Date of filing: 24.05.2017
(51) Int. Cl.: G06Q 20/40, G07C 9/00, H04L 29/06, G06F 19/00, G06Q 50/24

(54) **AUTHENTICATION PLATFORM AND METHOD**

(71) Applicant: Mastercard International Incorporated, Purchase, New York 10577 (US)
(72) Inventor: CUMMINS, Edwin, Dublin, 6 (IE); MONAGHAN, Karl, Dublin, 7 (IE); WHELAN, Darragh, Dublin, 8 (IE); DIXON, Conor, Dublin, 14 (IE); O'BRIEN, Gregory, Dublin (IE)
(74) Representative: Lawrence, Richard Anthony

(57) **Abstract**

A method of authenticating a user with a computing device associated with a camera is described. The computing device obtains first data from a token held by the user, and then captures a user image with the camera. The first data and the captured user image are then sent to an authentication server. If a record matches the first data, the record comprising the first data and a stored user image, the computing device receives information to indicate whether the user is authenticated. A suitable computing device is described, together with a suitable authentication server and an associated method carried out at the authentication server.

## Description

### Field of Disclosure

The present disclosure relates to an authentication platform and method. Embodiments of the disclosure are particularly relevant to dispensing of medication to individuals.

### Background of Disclosure

User authentication is well known in a wide variety of systems and is used for a user to gain access to accounts, entry to buildings, and for a wide range of other permissions and validations. Many methods are used including possession of a physical credential (such as a chip card), a virtual credential (such as a password or knowledge specific to the user) or a biometric identifier (such as a fingerprint). While user authentication is widely implemented, it can be more challenging in environments with poor computing and networking resources, or for users who have a very limited set of credentials or identifiers that can be conveniently used.

One area where user authentication is desirable, but challenging, is in dispensing of medication to widely dispersed populations in challenging environments - for example, in the dispensing of anti-retroviral drugs to combat the AIDS epidemic in sub-Saharan Africa. In 2016, it is estimated that of the population of South Africa, approximately 5.5 million (10%) have HIV/AIDS, with state-funded treatment being provided for about half that number. Reliable provision of state-funded treatment is problematic, because much provision is to the poorest (and so least well documented) in the community, and the least able to take a positive role in any user authentication process. Currently, states provide a card to individual claimants, and anti-retroviral drugs are provided to users providing the card to the medical service. This is problematic - there is widespread fraud through re-use and trading of cards, with the result that many users do not receive drugs that they have been allocated, with many issued drugs being resold on the black market.

It would be desirable to find a user authentication solution that would be effective for use in challenging environments of this kind while still providing reasonable certainty that the correct user had been authenticated. This would allow, for example, improved delivery of medication to an affected population, and improved effectiveness in the tracking of that delivery.

### Summary of Disclosure

In a first aspect, the disclosure provides a method of authenticating a user with a computing device associated with a camera, the method comprising the computing device: obtaining first data from a token held by the user; capturing a user image with the camera; sending the first data and the captured user image to a authentication server; and if a record matches the first data, the record comprising the first data and a stored user image, receiving information to indicate whether the user is authenticated.

The information to indicate whether the user is authenticated may comprise a confirmation that there is a record for the user and the captured user image matches one or more stored user images.

The information to indicate whether the user is authenticated may comprise a confirmation that there is a record for the user and one or more stored user images to allow a match to be determined at the computing apparatus. If no record matches the first data, the first data and the captured user image may be used to create a new record.

The method of authentication may be associated with provision of a service by a provider, the provider controlling the computing apparatus. This record may further comprise a history of provision of the service. Some or all of the history may be provided to the computing device on authentication of the user. The service may comprise providing medication to users.

In a second aspect, the disclosure provides a computing device comprising a processor and a memory and having a camera associated therewith, wherein the computing device is programmed to perform the method described above.

In a third aspect, the disclosure provides a method of authenticating a user at an authentication server from data received from a computing device associated with a camera, the method comprising the authentication server: receiving first data, comprising user identity data, and a captured user image from a computing device; determining whether a record in a user database matches the user identity data, and retrieving one or more stored user images associated with the record; and providing information to the computing device to indicate whether the user is authenticated.

The authentication server may match the captured user image against the one or more stored user images, and the information to indicate whether the user is authenticated may comprise a confirmation that there is a record for the user and the captured user image matches one or more stored user images.

The information to indicate whether the user is authenticated may comprise a confirmation that there is a record for the user and one or more stored user images to allow a match to be determined at the computing apparatus.

If no record matches the first data, the first data and the captured user image may be used to create a new record.

The method of authentication may be associated with provision of a service by a provider, and wherein the record further may comprise a history of provision of the service, with the authentication server providing some or all of the history to the computing device on authentication of the user.

In a fourth aspect, the disclosure provides an authentication server comprising a processor and a memory and comprising a database having a plurality of records therein, wherein a record comprises user identity data and one or more stored user images, wherein the authentication server is programmed to perform the method set out above.

### Brief Description of Figures

Embodiments of the disclosure will now be described, by way of example, with reference to the accompanying Figures, of which:
Figure 1 shows the elements of a system adapted to implement embodiments of the disclosure;
Figures 2A and 2B illustrate a computing device and an authentication server suitable for use in implementing embodiments of the disclosure;
Figure 3 is a flow diagram indicating a method according to an embodiment of the disclosure; and
Figures 4A to 4F illustrate pictorially interactions in a system adapted to implement the method of Figure 3.

### Description of Specific Embodiments

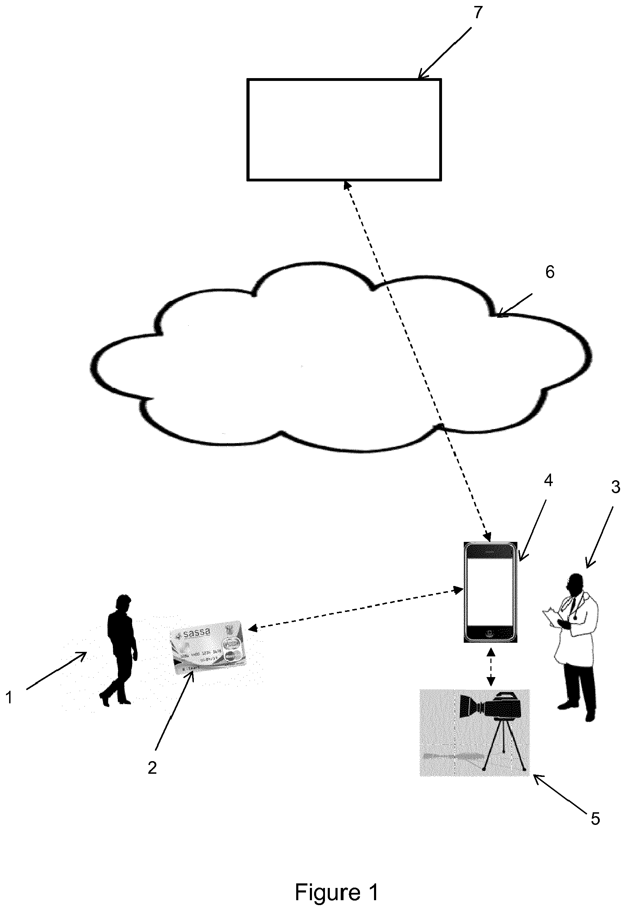

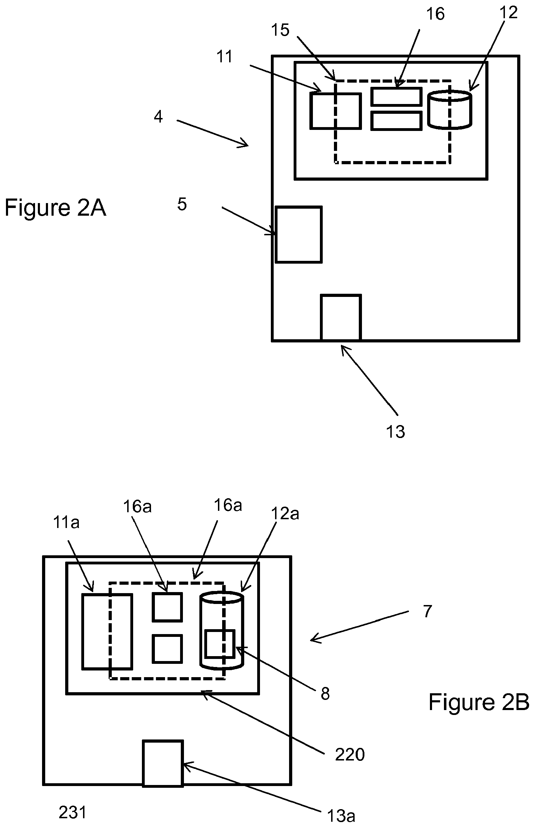

Specific embodiments of the invention will be described below with reference to the Figures. Figure 1 shows elements of a system adapted to implement an embodiment of the disclosure in connection with the distribution of medication. A user 1 of the medication system is equipped with a token 2 holding a user credential. This token 2 may be a government identity card, as here, or may be a transaction card or passport. The user 1 presents the token 2 to a provider 3. The provider 3 has a computing device 4 that is adapted to read the credential 2 and which also comprises or is associated with a camera 5 adapted to capture an image of the user 1. The computing device 4, as is shown schematically in Figure 2A, comprises in addition to the camera 5 (shown in this case as integral to the computing device, though as shown in Figure 1 it may also be a separate object but associated - for example, by a short range wireless networking technology such as Bluetooth) at least a processor 11, a memory 12, and networking capability 13, may be for example a cellular telephone handset or a tablet computer. The processor 11 and memory 12 between them define a computing environment 15 in which one or more applications 16 may run. The networking capability 13 will allow access to a network such as the public Internet, for example through a cellular telephone network or a wireless network.

The computing device 4 connects through the public Internet 6 or otherwise to a authentication server 7. The authentication server, as shown in Figure 2B, comprises at least a processor 11 a, a memory 12a, and networking capability 13a, with the memory 12a having a database 8 stored therein. The processor 11 a and memory 12a between them define a computing environment 15a in which one or more applications 16a may run. As will be discussed below, suitable software applications run in the computing device 4 and the authentication server 7 to implement embodiments of the disclosure.

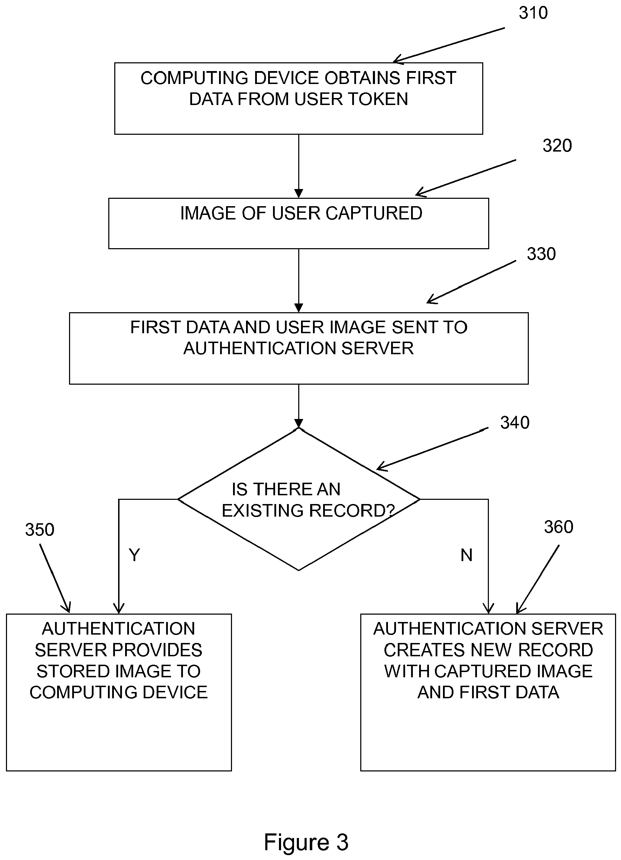

Figure 3 is a flow diagram illustrating a method of authenticating a user according to an embodiment of the disclosure. The method involves the use of the computing device 4 and its associated camera 5.

First of all the computing device obtains 310 first data from a token held by the user. The token will typically be a card associated with the user - for example an identity card, or a transaction card - and the first data will be a credential associated with the card (such as a PAN number). As discussed below, the first data may be provided in more than one way, and may be provided in such a way that the computing device 4 or its controller may be confident that the token is legitimate and that the first data is properly associated with the token.

After this, an image of the user is captured 320 with the camera 5 associated with the computing device 4. The computing device 4 then has both the first data and the captured user image, and sends 330 both to the authentication server.

The authentication server determines 340 whether or not there is a record for that first data, with a record comprising the first data and a stored user image. If there is such a record, then the authentication server provides 350 the computing device with the stored image to allow the controller of the computing device to determine whether the person physically in control of the token is the user associated with the token - the authentication server may also provide additional information associated with the record at this time. If there is no such record, then the authentication server creates 360 one with the first data and the captured user image.

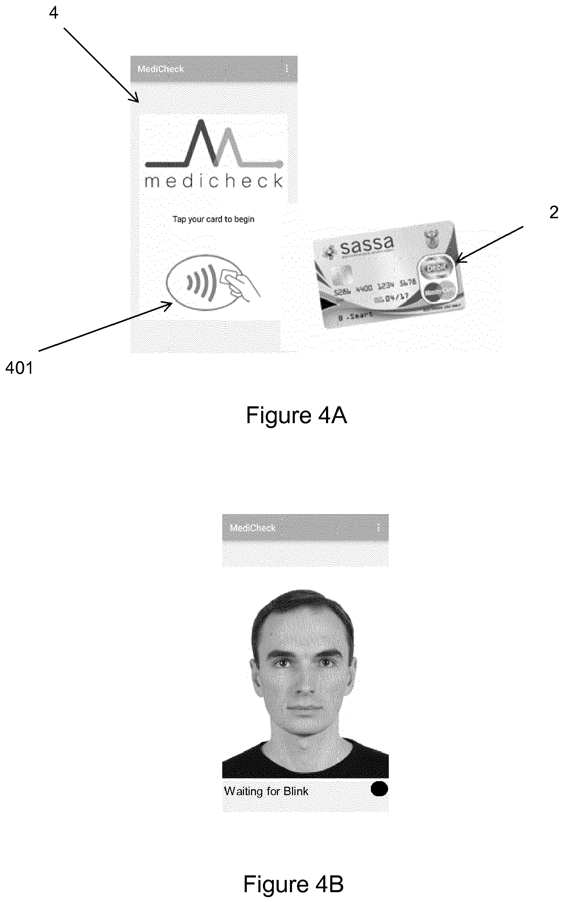

These steps and the associated system will now be described in more detail in the context of providing medication to users with reference to Figures 4A to 4F, which show the interactions at each step of the procedure. As discussed earlier, distribution of medications such as anti-retrovirals for state-funded treatment is problematic, because much provision is to those least able to take a positive role in any user authentication process. Currently, states provide a card to individual claimants - such as the South African SASSA card or the Nigerian eID - and anti-retroviral drugs are provided to users providing the card to the medical service. The embodiment described is directed to providing a user authentication solution that enables effective distribution of medication to this population while still providing reasonable certainty that the correct user had been authenticated.

First of all, the user is provided with a token of some kind that provides some type of user credential. This could be an existing identity card (such as the SASSA card or the eID) dispensed by the relevant authority in the conventional manner, or a dedicated ID card for this particular purpose. Alternatively, it could be a payment card (such as a conventional debit card or payment card), or some other type of chip card. Typically, such cards will implement the ISO/IEC 7810 standards for identification cards. In other embodiments, the token may be a wearable device (for example, a tag or other chip in a wristband or item of clothing). As will be discussed below, in embodiments the token is adapted for short range wireless interaction with a computing device using an appropriate protocol, generally based on or compatible with the ISO/IEC 14443 standard (for example, Near Field Communication protocols or the EMV contactless payment protocols). Other embodiments may use a different approach - the first data may be provided as magnetic stripe data, in a glyph such as a 2D or 3D barcode, or even simply as numbers and letters (which may, for example, be read by optical character recognition at the computing device).

The user credential (first data item) is an item of data that is useful as a persistent reference to the user. In embodiments, this may be a data item that is not visible on the token, but provided only as digital data - in other embodiments, it may be both (such as the PAN of a transaction card). Embodiments in which the first data is not provided as digital data may also be used. However, the embodiments that are discussed in detail below use digital data exchanged by a contactless protocol.

The computing device may be any appropriate to the context, but may most conveniently be a mobile telephone with an appropriate application (in this case termed MedCheck) installed on it. The Figure 4A to 4F examples show a user screen of a mobile telephone handset with the MedCheck application installed and running on it.

As shown in Figure 4A, the first data is provided to the computing device from the token, in this case by a contactless interaction between the token 2 and the computing device 4 in accordance with an ISO/IEC 14443 compliant standard. Typically, the provider will start an application running on the computing device which waits for contactless interaction with the token to progress (the relevant waiting screen being that shown in Figure 4A - the standard sign 401 for contactless interaction is shown on this screen). The specific standard used in the embodiment described below is that employed for EMV contactless payment (for which specifications are generally available from EMVCo at https://www.emvco.com/specifications.aspx). In this case, a conventional contactless interaction between the token and the computing device takes place according to existing EMV protocols, in the course of which various items of data are provided from the token to the computing device.

As noted, the data item or items provided may be any item appropriate to identify the user persistently and so to be appropriate to reference the user record in the authentication server. This could be a clearly visible identifier such as the Primary Account Number (PAN) for a card configured according to an EMV transaction card model, or other identification information, possibly in a repurposed EMV protocol field or stored in any appropriate protocol for storage of electronic identification information, such as ICAO 9303 for machine readable travel documents.

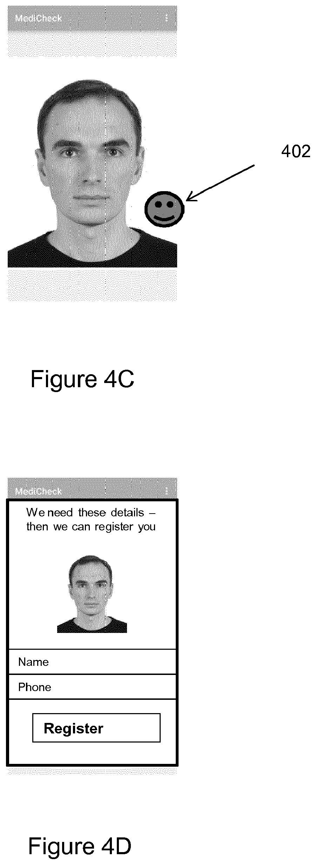

As shown in Figure 4B, the next step is for an image of the user to be captured with the camera in or associated with the computing device - this will typically be the camera of a phone, but may be any other type of camera in communication with the computing device. The provider will in any event have been in control of the capture of the user image through the application running on the provider computing device, so the provider can be confident that the first data has been obtained from the user token and that the captured image is of the user physically bearing the token. In the case indicated in Figure 4B, the image is captured through a phone camera, with image capture triggered by the user blinking - the person skilled in the art will appreciate that a blink is an easily recognised gesture and code for blink recognition can be written according to well known principles or obtained from existing sources. When a satisfactory image is captured, and appropriate symbol 402 is provided by the application, as shown in Figure 4C.

The first data and the captured image are then sent to the authentication server by the application. This could be by any appropriate network communication route, and may in some environments include mutual authentication of the computing device and the authentication server (or the relevant applications) and establishment of a secure channel for information. Use of a financial transaction infrastructure may be used in some embodiments, accessed for example by an internet gateway.

The first data is used to determine whether there is a record for the relevant user in the database. As discussed previously, different paths will be followed depending on whether or not a record exists.

Figure 4D shows a case where a user record does not exist. The authentication server communicates back that there is no existing record, and asks the user to register so that a record can be established. If the user and provider agree to do this, the user will enter registration details as shown in the captured screen, and a record will then be established using the captured user image as a new stored user image.

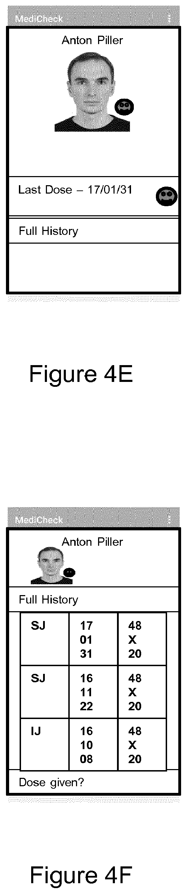

Figure 4E shows a result if there is a record for the user on the system. One section 403 of the screen show details of the user record. In the approach shown, image recognition takes place at the authentication server (or in another system associated with the authentication server), and a determination is made that the captured user image matches the existing user image (or images, as discussed below) - this is shown here by an indication 404 that this is an approved image. In an alternative approach, there may be no recognition at the authentication server, but instead the authentication server returns any images that it has to allow the provider to make a face-to-face determination that there is a match. A second section of the screen indicates the last dose date 405 for that user, together with an indication 406 that this means that further medication may be provided - again, in an alternative approach, the decision to provide further medication may be left to the provider, with the application only providing the history information. A third section of the screen provides a full history tab 407.

The expansion of the full history tab is shown in Figure 4F. This lists previous interactions of the user with providers, indicating provider, dose and date. The provider action (to provide medication, or to refuse to provide medication) may then be recorded and sent to the authentication server to add to the user record. Details of the interaction that takes place between the user and provider - for example, identification of medicaments given by the provider to the user and the date of transfer - would then be recorded at the computing device and passed to the authentication server to add to the user record. If this approach is used, the user record is not only used for authentication, but also to provide an interaction history between provider and user (which may be particularly useful if there are multiple providers). In other embodiments, however, a full history may not be offered to the provider in this way, but only an indication that medication should or should not be dispensed - the information recorded in the MedCheck application may be a single button press to indicate that medication was provided to that user.

Alternative approaches are possible for user images, as there is a new user image captured on each interaction - the original image stored when the record was established may be provided to the MedCheck application, but subsequent images provided during earlier authentication stages may also be provided to allow greater certainty of matching (though this may need to be balanced against memory considerations). This may be particularly helpful in embodimets where the provider is asked to confirm whether the provider accepts the captured image as a match with the existing user images, rather than user image matching taking place in the authentication server.

While this approach may be used to provide a convenient and low-cost approach to providing medication to a poorly documented community, it has other potential applications. It may, for example, be used to provide confirmation that the user is able to make certain kinds of transaction (for example, that the user is old enough to purchase alcohol) or to allow permission to the user to access a controlled site or system. The skilled person will appreciate that the embodiments described here are exemplary, and that modifications may be made and alternative embodiments provided that fall within the scope of the disclosure.

## Claims

1. A method of authenticating a user with a computing device associated with a camera, the method comprising the computing device:
obtaining (310) first data from a token held by the user;
capturing (320) a user image with the camera;
sending (330) the first data and the captured user image to a authentication server; and
if a record matches the first data, the record comprising the first data and a stored user image, receiving information to indicate whether the user is authenticated.

2. The method of claim 1, wherein the information to indicate whether the user is authenticated comprises a confirmation that there is a record for the user and the captured user image matches one or more stored user images.

3. The method of claim 1, wherein the information to indicate whether the user is authenticated comprises a confirmation that there is a record for the user and one or more stored user images to allow a match to be determined at the computing apparatus.

4. The method of claim 1, wherein if no record matches the first data, the first data and the captured user image are used to create a new record.

5. The method of any preceding claim, wherein the method of authentication is associated with provision of a service by a provider, the provider controlling the computing apparatus.

6. The method of claim 5, wherein the record further comprises a history of provision of the service.

7. The method of claim 6, wherein some or all of the history is provided to the computing device on authentication of the user.

8. The method of any of claims 5 to 7, wherein the service comprises providing medication to users.

9. A computing device comprising a processor and a memory and having a camera associated therewith, wherein the computing device is programmed to perform the method of any of claims 1 to 8.

10. A method of authenticating a user at an authentication server from data received from a computing device associated with a camera, the method comprising the authentication server:
receiving (330) first data, comprising user identity data, and a captured user image from a computing device;
determining (340) whether a record in a user database matches the user identity data, and retrieving one or more stored user images associated with the record; and
providing (350) information to the computing device to indicate whether the user is authenticated.

11. The method of claim 10, further comprising the authentication server matching the captured user image against the one or more stored user images, and wherein the information to indicate whether the user is authenticated comprises a confirmation that there is a record for the user and the captured user image matches one or more stored user images.

12. The method of claim 10, wherein the information to indicate whether the user is authenticated comprises a confirmation that there is a record for the user and one or more stored user images to allow a match to be determined at the computing apparatus.

13. The method of claim 10, wherein if no record matches the first data, the first data and the captured user image are used to create a new record.

14. The method of any of claims 10 to 13, wherein the method of authentication is associated with provision of a service by a provider, and wherein the record further comprises a history of provision of the service, further comprising the authentication server providing some or all of the history to the computing device on authentication of the user.

15. An authentication server comprising a processor and a memory and comprising a database having a plurality of records therein, wherein a record comprises user identity data and one or more stored user images, wherein the authentication server is programmed to perform the method of any of claims 10 to 14.
